# EUROPÄISCHE PATENTSCHRIFT

(11) **EP 1 321 947 B1**
(45) Veröffentlichungstag und Bekanntmachung des Hinweises auf die Patenterteilung: **03.11.2010**
(21) Anmeldenummer: 02025887.7
(22) Anmeldetag: 19.11.2002
(51) Int. Cl.: G21F 3/03

(54) **Strahlenschutzbekleidung mit einer separaten Umhüllung**
Radiation protection garment having a separate cover
Vêtement protecteur contre les radiations ayant une enveloppe séparée

(30) Priorität: 19.12.2001 DE 10162594
(43) Veröffentlichungstag der Anmeldung: 25.06.2003
(73) Patentinhaber: Mavig GmbH, 81829 München (DE)
(72) Erfinder: Ballsieper, Barbara, 82024 Taufkirchen (DE)
(74) Vertreter: Körfer, Thomas

(56) Entgegenhaltungen:
- DE-U1- 8 705 533
- US-A- 5 015 865
- US-A- 5 073 984
- US-A- 5 115 140
- US-A- 5 523 581

## Beschreibung

Die Erfindung betrifft eine Strahlenschutzbekleidung, insbesondere eine Strahlenschutzbekleidung zur Abschirmung der durch eine Röntgenstrahlungsquelle abgestrahlte Röntgenstrahlung, mit einer separaten Umhüllung gemäß Anspruch 1.

Aus der US 4,196,355 ist eine zweiteilige Strahlenschutzbekleidung bekannt, welche eine Weste zum Schutz des Oberkörpers und einen Rock zum Schutz des Unterkörpers umfaßt. Die Weste und der Rock bestehen aus mehreren inneren Lagen eines flexiblen Strahlenschutzmaterials, welche mit einem nicht schützenden Stoff oder ähnlichem oder einem Material wie Vinyl etc. überzogen sind.

Nachteilig bei der aus der US 4,196,355 bekannten Strahlenschutzbekleidung ist insbesondere, daß die das Strahlenschutzmaterial umgebende Stoff- oder Vinyllage fest mit der Strahlenschutzbekleidung verbunden ist und nicht ohne großen Aufwand vom Strahlenschutzmaterial getrennt werden kann. Dies ist besonders im operativen Anwendungsbereich, beispielsweise in Kliniken, von Nachteil, da über der Strahlenschutzbekleidung jeweils ein weiteres, steriles Bekleidungsstück getragen werden muß, weil die Strahlenschutzbekleidung nicht sterilisierbar ist.

US 5,523,581 A zeigt ein Strahlungsabschirmungssystem für medizinische Prozeduren, das ein flexibles Schild aus strahlungsabschwächendem Material umfasst. Das flexible Schild besteht aus einem viskoelastischen Material, das strahlungsabschwächendes Füllmaterial enthält, wobei das flexible Schild permanent in einem Gehäuse gehalten ist, um es einfach platzieren zu können.

US 5,115,140 A offenbart ein Bekleidungsstück, welches seinen Benutzer gegenüber elektromagnetischen bzw. Strahlungsfrequenzen oder gegenüber Mikrowellenstrahlung, die von elektronischen Geräten ausgeht, abschirmt. Das Bekleidungsstück enthält eine Außenhaut aus biegsamem Material mit einer flexiblen Einlage, die eine aus Kupfer basierende Beschichtung aufweist.

DE 87 05 533 U beschreibt ein Kleidungsstück zum Schutz gegen radioaktive Strahlung mit einem Vorderteil, einem Schulterteil zum Aufhängen des Kleidungsstücks zum Aufhängen am Körper eines Benutzers und einen um das Kleidungsstück herum auf Hüft- bzw. Taillenhöhe anbringbaren elastischem Gürtel, der um zumindest einen Teil der Taille so festziehbar ist, dass zumindest ein Teil des Gewichts des Kleidungsstücks von den Hüften und/oder der Taille getragen wird.

US 5,073,984 zeigt eine personengebundene Schutzkleidung in Form einer Weste, eines Rockes, einer Kapuze, eines Mantels oder ähnlichem, wobei eine verwebt oder gestrickte Faser, dessen Oberfläche metallisiert ist, verwendet wird.

Um die von einer Röntgenquelle ausgehenden Strahlungen gegenüber einem Körper ausreichend abzuschirmen, gibt die Röntgenverordnung mittels der DIN 6813 einen minimalen Bleigleichwert von 0,35 mm im Frontbereich und 0,25 mm im Rückenbereich einer Röntgenschutzbekleidung vor. Im Hinblick auf die hohen Belastungen eines Arztes, der täglich einer Röntgenbestrahlung ausgesetzt ist, ist ein Bleigleichwert von 0,5 mm im Frontbereich vorteilhaft. Dabei wird mit dem Bleigleichwert das Absorptionsverhalten eines Körpers, insbesondere eines Laminates beschrieben, das gegenüber Röntgenstrahlung eine Abschirmung aufweist, wie sie eine Bleiplatte der entsprechenden Dicke hat. Ein Material mit einem Bleigleichwert von 0,25 mm entspricht daher einer Bleiabschirmung mit einer Stärke von 0,25 mm.

Aufgabe der Erfindung ist es daher, eine Strahlenschutzbekleidung zu schaffen, die den erforderlichen Schutz vor Strahlung, insbesondere vor Röntgenstrahlung, gewährleistet, bequemes und sicheres Tragen und ausreichende Bewegungsfreiheit bietet und gleichzeitig in einfacher Weise für die Anwendung im operativen Bereich steril gehalten werden kann.

Die Aufgabe wird durch eine erfindungsgemäße Strahlenschutzbekleidung mit den Merkmalen des Anspruchs 1 gelöst. Vorteilhafterweise ist dabei das eigentliche Strahlenschutzbekleidungsstück durch eine etwa gleich geformte Umhüllung umgeben, welche das Strahlenschutzbekleidungsstück vollständig umgibt. Dadurch wird gewährleistet, daß die selbst nur schwer sterilisierbaren Strahlenschutzbekleidungsstücke in einer sterilen Umgebung wie z. B. einem Operationssaal verwendet werden können, ohne daß aufwendige und teure Sterilisierungsmaßnahmen für das empfindliche Strahlenschutzmaterial durchgeführt werden müssen.

Erfindungsgemäß ist das Strahlenschutzbekleidungsstück mit der Umhüllung lösbar verbunden, wobei die lösbare Verbindung des Strahlenschutzbekleidungsstücks mit der Umhüllung aus komplementären Klettverschlüssen besteht. Die verschiedenen Klettverschlussteile sind erfindungsgemäß mittels Druckknöpfen oder Reißverschlüssen austauschbar an der Umhüllung als auch an dem Strahlenschutzbekleidungsstück fixiert.

Durch die in den Unteransprüchen aufgeführten Maßnahmen sind vorteilhafte Weiterbildungen der im Anspruch 1 angegebenen Strahlenschutzbekleidung möglich.

Vorteilhaft ist es, daß die Umhüllung zumindest eine durch ein Verschlußsystem wiederverschließbare Öffnung aufweist, durch welche das Strahlenschutzbekleidungsstück in die Umhüllung eingelegt werden kann.

Das Verschlußsystem der Öffnung kann dabei vorteilhafterweise aus Klettverschlüssen, Reißverschlüssen oder Druckknöpfen bestehen.

Die Umhüllung besteht dabei aus einem Material, welches wie herkömmliche Operationskittel in einer geeigneten Vorrichtung wie z. B. durch Kochen oder in einem Autoklav sterilisiert werden kann.

Ausführungsbeispiele der Erfindung sind in den nachfolgenden Zeichnungen vereinfacht dargestellt und in der nachfolgenden Beschreibung näher erläutert. Es zeigen:
- Fig. 1A: ein erstes Ausführungsbeispiel einer erfindungsgemäßen Strahlenschutzbekleidung bei Gebrauch;
- Fig. 1B: eine schematische Darstellung der Umhüllung und des Strahlenschutzbekleidungsstücks der in Fig. 1A dargestellten Strahlenschutzbekleidung;
- Fig. 2A: ein zweites Ausführungsbeispiel einer erfindungsgemäßen Strahlenschutzbekleidung bei Gebrauch; und
- Fig. 2B: eine schematische Darstellung der Umhüllung und des Strahlenschutzbekleidungsstücks der in Fig. 2A dargestellten Strahlenschutzbekleidung.

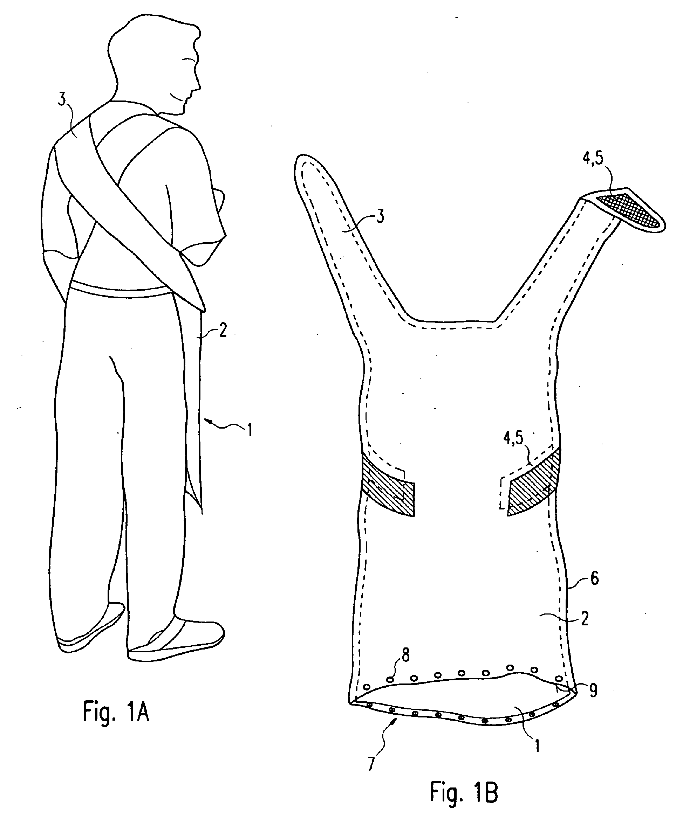

Fig. 1A zeigt ein Ausführungsbeispiel einer erfindungsgemäßen Strahlenschutzbekleidung 1 in angelegtem Zustand. Die erfindungsgemäße Strahlenschutzbekleidung 1 eignet sich zur Abschirmung einer insbesondere von einer Röntgenquelle abgestrahlten Strahlung, wie sie z.B. im medizinischen Bereich, insbesondere in der radiologisch gestützten Endoskopie, Verwendung findet. Die erfindungsgemäße Strahlenschutzbekleidung 1 eignet sich jedoch auch für andere Anwendungsfälle.

Die im vorliegenden ersten Ausführungsbeispiel dargestellte Strahlenschutzbekleidung 1 wird mittels zweier Befestigungsbänder 3 fixiert, welche hinter dem Rücken der die Strahlenschutzbekleidung 1 tragenden Person gekreuzt werden und mittels geeigneter Verschlüsse 4, beispielsweise Klettverschlüsse oder Schnallenverschlüsse, verbunden werden. Die einzelnen Teile der Verschlüsse 4 sind dabei in entsprechenden Positionen der Strahlenschutzbekleidung 1 angeordnet. Beispielsweise können Klettverschlußbänder 5 auf der Strahlenschutzbekleidung 1 in Hüfthöhe fixiert sein und mit entsprechenden komplementären Klettverschlußbändern 5 an den Enden der Befestigungsbänder 3 korrespondieren, so daß eine Fixierung der Strahlenschutzbekleidung 1 durch das zumindest teilweise Aufeinanderlegen der komplementären Klettverschlußbänder 5 erfolgen kann. Die Strahlenschutzbekleidung 1 erstreckt sich dabei bis in die Knieregion der die Strahlenschutzbekleidung 1 tragenden Person und weist beispielsweise einen Bleigleichwert von 0,5 mm auf. Die Befestigungsbänder 3 tragen dabei in diesem Ausführungsbeispiel nicht zum Strahlenschutz bei.

Um zu gewährleisten, daß mit der Strahlenschutzbekleidung 1 keine Keime beispielsweise in die sterile Umgebung eines Operationssaales eingeschleppt werden, ist es bisher üblich, über der Strahlenschutzbekleidung 1 einen sterilen Operationskittel zu tragen. Dieser wird gewöhnlich durch Kochen oder mittels eines Autoklav oder einer Bedampfung unter Schutzatmosphäre mit einem entsprechenden Gasgemisch sterilisiert, während die Strahlenschutzbekleidung 1 nur oberflächlich gereinigt und mit einem Desinfektionsmittel abgerieben werden kann. Die Sterilisierung von Strahlenschutzbekleidung 1 kann nur unter großem Aufwand geschehen, da die Strahlenschutzbekleidung 1 durch hohe Temperaturen beschädigt wird, wodurch die Schutzfunktion nicht mehr zuverlässig gewährleistet ist.

Daher wird erfindungsgemäß vorgeschlagen, ein beliebiges Strahlenschutzbekleidungsstück 2 in eine sterile Umhüllung 6 einzulegen, welche in Fig. 1B schematisiert dargestellt ist. Die Umhüllung 6 ist dabei ungefähr so geformt wie das zu umhüllende Strahlenschutzbekleidungsstück 2. Sie weist zumindest eine verschließbare Öffnung 7 auf, durch welche das Strahlenschutzbekleidungsstück 1 in die Umhüllung 6 eingelegt werden kann. Das herkömmliche Strahlenschutzbekleidungsstück 2 braucht für die Anwendung mit einer erfindungsgemäßen Umhüllung 6 nicht wesentlich umgestaltet zu werden.

Die Umhüllung 6 weist an der zumindest einen verschließbaren Öffnung 7 ein Verschlußsystem 8 auf, durch welches die Umhüllung 6 möglichst dicht verschließbar ist. Im vorliegenden Ausführungsbeispiel ist die Öffnung 7 an einer unteren Kante 9 der Umhüllung 6 beispielhaft in Form von Druckknöpfen 8 ausgebildet. Als Verschlußsystem 8 bieten sich bei einer derartigen Anordnung weiterhin ein Reißverschluß oder Klettverschlüsse an. Wenn die Umhüllung 6 etwas länger als die Strahlenschutzbekleidung 1 ist, kann das Verschlußsystem u. U. gänzlich wegfallen, was die Herstellung, die Handhabung und die Sterilisation der Umhüllung 6 vereinfacht.

Für größere Strahlenschutzbekleidungsstücke 2 mit unterschiedlichen Schnitten ist es hilfreich, die Umhüllung 6 mit dem Strahlenschutzbekleidungsstück 2 zu verbinden. Auch hierbei sind Klettverschlußteile, welche an zweckdienlichen Stellen angebracht werden, von Vorteil. Bekannt sind ebenso einzelne Druckknöpfe oder Schnallenverschlüsse.

Die Strahlenschutzbekleidung 1 mit der Umhüllung 6 wird in gleicher Weise fixiert wie das Strahlenschutzbekleidungsstück 2 alleine. Daher weist die Umhüllung an gleicher Stelle die entsprechenden Verschlüsse 4 wie das Strahlenschutzbekleidungsstück 2 auf. Erfindungsgemäß werden jeweils komplementäre Klettverschlüsse 5 gewählt. Die gestrichelt in Fig. 1B angedeuteten Klettverschlußteile 5 gehören dabei zu dem Strahlenschutzbekleidungsstück 2, während die schraffierten Klettverschlußteile 5 auf der Umhüllung 6 angebracht sind. Die verschiedenen Klettverschlußteile 5 sind dabei mittels nicht näher dargestellter Druckknöpfe oder Reißverschlüsse austauschbar an der Umhüllung 6 wie auch an dem Strahlenschutzbekleidungsstück 2 fixiert.

Die komplementären Klettverschlußteile 5 sind an den Enden der Befestigungsbänder 3 ebenfalls an gleicher Stelle wie an dem Strahlenschutzbekleidungsstück 2 selbst angebracht, so daß die in der Umhüllung 6 verpackte Strahlenschutzbekleidung 1 in gleicher Weise angelegt und fixiert werden kann wie das Strahlenschutzbekleidungsstück 2 alleine.

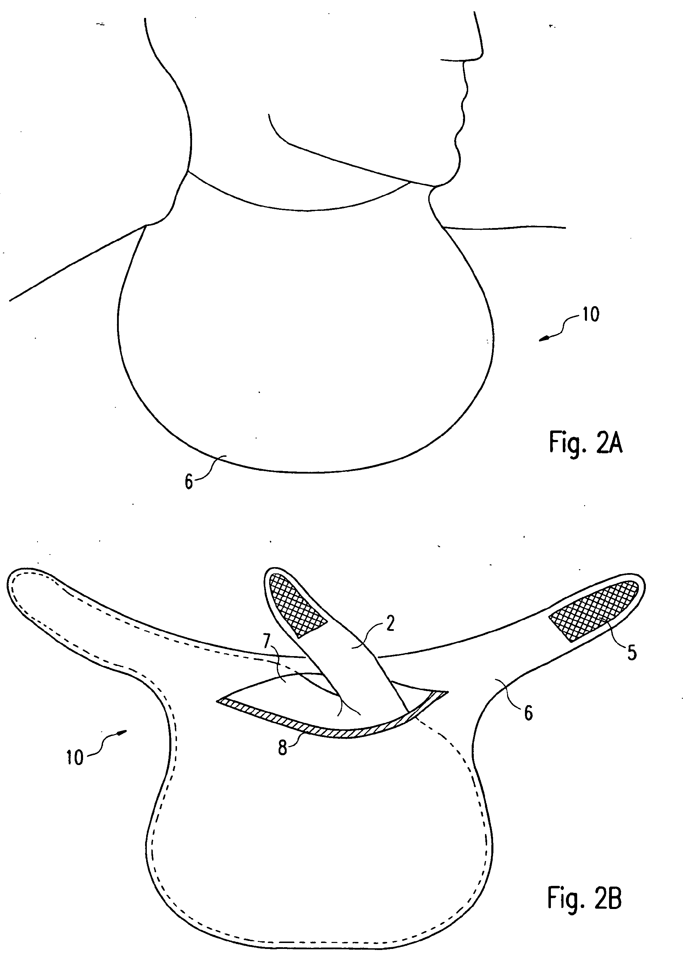

Fig. 2A zeigt in einer schematischen Ansicht ein zweites Ausführungsbeispiel für eine erfindungsgemäß ausgestaltete Strahlenschutzbekleidung 1. In diesem Ausführungsbeispiel ist ein sog. Sternumschutz 10, welcher die Schilddrüsen- und Brustbeinregion schützt, dargestellt.

Fig. 2B zeigt eine schematische Ansicht des entsprechenden Strahlenschutzbekleidungsstücks 2, welches teilweise in eine passend geformte Umhüllung 6 eingesteckt ist. Durch die handliche Größe des Sternumschutzes 10 ist es relativ einfach, das Strahlenschutzbekleidungsstück 2 in die Umhüllung 6 einzulegen. Die Öffnung 7 kann dabei vorteilhafterweise dem Körper der den Sternumschutz 10 tragenden Person zugewandt sein, so daß die gesamte dem Körper abgewandte Seite steril bleibt und nicht mit dem nicht sterilen Strahlenschutzbekleidungsstück 2 in Berührung kommt.

Die Klettverschlußteile 5, welche an der Umhüllung 6 angeordnet sind, sind ebenso wie bei der in der in Fig. 1A und 1B dargestellten Strahlenschutzbekleidung 1 an den gleichen Stellen angebracht wie an dem Strahlenschutzbekleidungsstück 2 selbst, so daß die Fixierung und damit die Paßform der Strahlenschutzbekleidung 1 gegenüber dem Strahlenschutzbekleidungsstück 2 identisch ist.

Die Erfindung ist nicht auf die beschriebenen Ausführungsbeispiele beschränkt. Insbesondere eignet sich die Erfindung für beliebige Zuschnitte der Strahlenschutzbekleidungsstücke 2.

## Patentansprüche

1. Strahlenschutzbekleidung (1) zur Abschirmung einer von einer Röntgenquelle abgestrahlten Strahlung aufweisend ein aus einem Strahlenschutzmaterial bestehendes Strahlenschutzbekleidungsstück (2) und eine das Strahlenschutzbekleidungsstück (2) vollständig umgebende Umhüllung (6), wobei das Strahlenschutzbekleidungsstück (2) in die Umhüllung (6) einlegbar und die Umhüllung (6) vollständig von dem Strahlenschutzbekleidungsstück (2) separierbar ist, wobei eine lösbare Verbindung des Strahlenschutzbekleidungsstücks (2) mit der Umhüllung (6) vorgesehen ist,
**dadurch gekennzeichnet,**
**dass** die lösbare Verbindung mittels komplementärer Klettverschlussteile (5) erfolgt, wobei die verschiedenen Klettverschlussteile (5) mittels Druckknöpfen oder Reißverschlüssen austauschbar an der Umhüllung (6) als auch an dem Strahlenschutzbekleidungsstück (2) fixiert sind.

2. Strahlenschutzbekleidung nach Anspruch 1,
**dadurch gekennzeichnet,**
**dass** jeweils eines der Klettverschlussteile (5) an der Umhüllung (6) und an dem Strahlenschutzbekleidungsstück (2) fixiert ist.

3. Strahlenschutzbekleidung nach einem der Ansprüche 1 oder 2,
**dadurch gekennzeichnet,**
**dass** die Umhüllung (6) zumindest eine Öffnung (7) aufweist, durch welche das Strahlenschutzbekleidungsstück (2) in die Umhüllung (6) einbringbar ist.

4. Strahlenschutzbekleidung nach Anspruch 3,
**dadurch gekennzeichnet,**
**dass** die zumindest eine Öffnung (7) der Umhüllung (6) mittels eines Verschlusssystems (8) verschlossen ist.

5. Strahlenschutzbekleidung nach Anspruch 4,
**dadurch gekennzeichnet,**
**dass** das Verschlusssystem (8) aus komplementären Klettbändern besteht.

6. Strahlenschutzbekleidung nach Anspruch 4,
**dadurch gekennzeichnet,**
**dass** das Verschlusssystem (8) aus Druckknöpfen besteht.

7. Strahlenschutzbekleidung nach Anspruch 4,
**dadurch gekennzeichnet,**
**dass** das Verschlusssystem (8) aus einem Reißverschluss besteht.

8. Strahlenschutzbekleidung nach einem der Ansprüche 1 bis 7,
**dadurch gekennzeichnet,**
**dass** die Umhüllung (6) aus einem mittels einer geeigneten Vorrichtung oder eines geeigneten Verfahrens sterilisierbaren Material besteht.

## Claims

1. Radiation-protection clothing (1) for shielding from radiation emitted from an x-ray source comprising an item of radiation-protection clothing (2) made from a radiation-protection material and a covering (6) completely surrounding the item of radiation-protection clothing (2), wherein the item of radiation-protection clothing (2) can be inserted into the covering (6), and the covering (6) can be completely separated from the item of radiation-protection clothing (2), wherein a detachable connection of the item of radiation-protection clothing (2) to the covering (6) is provided,
**characterised in that**
the detachable connection is implemented by means of complementary Velcro-type connecting parts (5), wherein the different Velcro-type connecting parts (5) are fixed in a replaceable manner by means of snap fasteners or zip fasteners to the covering (6) and also to the item of radiation-protection clothing (2).

2. The radiation-protection clothing according to claim 1,
**characterised in that**,
one of the Velcro-type connecting parts (5) is fixed respectively to the covering (6) and to the item of radiation-protection clothing (2).

3. The radiation-protection clothing according to any one of claims 1 or 2,
**characterised in that**
the covering (6) provides at least one opening (7), through which the item of radiation-protection clothing (2) can be introduced into the covering (6).

4. The radiation-protection clothing according to claim 3,
**characterised in that**
the at least one opening (7) of the covering (6) is closed by means of a closure system (8).

5. The radiation-protection clothing according to claim 4,
**characterised in that**
the closure system (8) comprises complementary Velcro-type strips.

6. The radiation-protection clothing according to claim 4,
**characterised in that**
the closure system (8) comprises snap fasteners.

7. The radiation-protection clothing according to claim 4,
**characterised in that**
the closure system (8) comprises a zip fastener.

8. The radiation-protection clothing according to any one of claims 1 to 7,
**characterised in that**
the covering (6) is made from a material capable of being sterilised by means of an appropriate device or an appropriate method.

## Revendications

1. Vêtement de protection (1) contre les radiations ou rayonnements destiné à former écran à l'encontre de radiations rayonnées par une source de rayons X, et comprenant une partie de vêtement de protection contre les radiations (2) réalisée en un matériau de protection contre les radiations, et une enveloppe (6) entourant complètement la partie de vêtement de protection contre les radiations (2), la partie de vêtement de protection contre les radiations (2) pouvant être insérée dans l'enveloppe (6) et l'enveloppe (6) pouvant être totalement séparée de la partie de vêtement de protection contre les radiations (2), une liaison amovible étant prévue entre la partie de vêtement de protection contre les radiations (2) et l'enveloppe (6), **caractérisé**
**en ce que** la liaison amovible s'effectue au moyen de pièces auto-agrippantes (5) complémentaires, les différentes pièces auto-agrippantes (5) étant fixées de manière interchangeable, au moyen de boutons-pression ou de fermetures à glissière, aussi bien sur l'enveloppe (6) que sur la partie de vêtement de protection contre les radiations (2).

2. Vêtement de protection contre les radiations selon la revendication 1,
**caractérisé en ce que** respectivement l'une des pièces auto-agrippantes (5) est fixée à l'enveloppe (6) et à la partie de vêtement de protection contre les radiations (2).

3. Vêtement de protection contre les radiations selon l'une des revendications 1 ou 2,
**caractérisé en ce que** l'enveloppe (6) présente au moins une ouverture (7) à travers laquelle la partie de vêtement de protection contre les radiations (2) peut être insérée dans l'enveloppe (6).

4. Vêtement de protection contre les radiations selon la revendication 3,
**caractérisé en ce que** ladite au moins une ouverture (7) de l'enveloppe (6) est fermée au moyen d'un système de fermeture (8).

5. Vêtement de protection contre les radiations selon la revendication 4,
**caractérisé en ce que** le système de fermeture (8) est constitué de bandes auto agrippantes complémentaires.

6. Vêtement de protection contre les radiations selon la revendication 4,
**caractérisé en ce que** le système de fermeture (8) est constitué de boutons-pression.

7. Vêtement de protection contre les radiations selon la revendication 4,
**caractérisé en ce que** le système de fermeture (8) est constitué d'une fermeture à glissière.

8. Vêtement de protection contre les radiations selon l'une des revendications 1 à 7,
**caractérisé en ce que** l'enveloppe (6) est réalisée en un matériau pouvant être stérilisé au moyen d'un dispositif approprié ou d'un procédé approprié.
